# EUROPEAN PATENT APPLICATION

(11) **EP 3 680 661 A1**
(43) Date of publication of application: **15.07.2020**
(21) Application number: 18854428.2
(22) Date of filing: 07.09.2018
(51) Int. Cl.: G01N 33/50, G01N 33/483, G01N 33/68

(54) **CUSTOMIZED GROWTH FACTOR SCREENING SYSTEM FOR CELL CULTURE**

(30) Priority: 07.09.2017 KR 20170114696
(71) Applicant: Samsung Life Public Welfare Foundation, Seoul 04348 (KR)
(72) Inventor: NAM, Do-Hyun, Seoul 06351 (KR); KANG, Hyun Ju, Seoul 06351 (KR); SHIN, Kayoung, Seoul 06351 (KR)
(74) Representative: Patentanwälte Olbricht Buchhold Keulertz
(86) International application number: PCT/KR2018/010458
(87) International publication number: WO 2019/050314

(57) **Abstract**

Provided is a method of screening a customized growth factor combination for cell culture, the method including: adding at least one growth factor and cells isolated from a subject to a culture medium; culturing the cells; and measuring cell proliferative activity of the cells. According to the method of screening cell-customized cell culture conditions according to one aspect, it is possible to efficiently select the optimal conditions for cell culture.

## Description

### TECHNICAL FIELD

The present application claims the priority of Korean Patent Application No. 10-2017-0114696, filed on September 7, 2017, and the entirety of which is a reference of the present application.

It relates to a customized growth factor screening system for tumor spheroid culture.

### BACKGROUND ART

*In vitro* biological research requires cell procurement. However, since it is difficult to implement, in cell culture media, conditions that are exactly the same as *in vivo* conditions, culture conditions (particularly culture media) capable of artificially promoting cell proliferation or maintaining cells have been applied to cell culture. In this case, various culture medium compositions are required according to the characteristics of the primary tissue of the cell, the *in vitro* culture environment, and the purpose of the culture, and in particular, the selection of a medium composition that can maximize cell culture efficiency and reflects the characteristics of primary cells is an essential element for *in vitro* cell culture.

In particular, as unmet clinical needs with respect to established cell lines emerge, the number of cases in which cells (tumor spheroids) collected from patients' disease sites are cultured and the cultured cells are used for patient-specific treatment and disease mechanism research has increased. Accordingly, the culturing of tumor spheroids has emerged as a necessary requirement. However, since there are differences in molecular genetic characteristics of individual patients and characteristics of primary tissues, it is difficult to select culture conditions in which cells isolated from the patient (particularly disease-specific cells only) can be grown/maintained. Under general culture conditions, 1) it is difficult to control the proliferation of cells other than the disease-specific cells, and thus, disease-specific cells in the cultured cells may not be cultured, and 2) cells of a specific group in the primary tissue only may be selected and cultured.

Therefore, there is a need to develop a method of screening culture conditions that can minimize the impact of these problems and maximize the culture efficiency of tumor spheroids.

### DESCRIPTION OF EMBODIMENTS

### TECHNICAL PROBLEM

Provided is a method of screening a customized growth factor combination for cell culture, the method including: adding at least one growth factor and cells isolated from a subject to a culture medium; culturing the cells; and measuring the cell proliferative activity of the cells.

### SOLUTION TO PROBLEM

An aspect provides a method of selecting a customized growth factor combination for cell culture, the method including: adding at least one growth factor and cells isolated from a subject to a culture medium; culturing the cells; and measuring the cell proliferative activity of the cells.

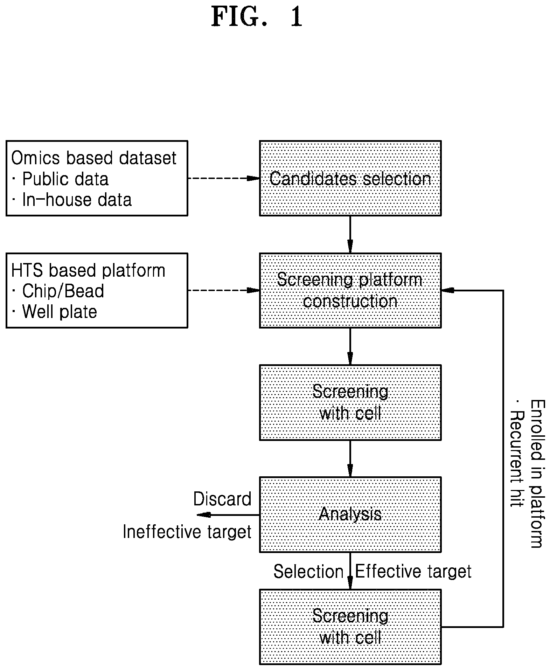

FIG. 1 is a schematic diagram showing a method of screening a customized growth factor combination for cell culture according to an aspect of the present disclosure.

First, any cell to be cultured may be selected. The cells may refer to tumor spheroids that need to be utilized for the purpose of medical research and treatment, commercialized cells that need to increase culture efficiency, or all cells that require cell culture. The cells may be sorted according to certain conditions as needed. The conditions may be the tissue from which cells are derived, the type of disease, the severity of the disease (for example, in the case of cancer, tumor grade, genotype, or other molecular-pathologic features).

According to the selected cells, the growth factor candidate groups to be added to the cell culture medium are classified from the omics-based database such as genome, transcriptome, and proteome. The database may be data which is published in a pathway accessible to those of ordinary skill in the art (for example, protein databases, http://www.proteinatlas.org/, http://www.uniprot.org/help/uniprotkb) or data accumulated by the skilled person in the laboratory. At this time, the selected candidate groups may be a growth factor set including two or more growth factors.

The selected candidate groups may be added simultaneously to dozens or thousands of media (wells, chips, beads) using a high-throughput screening (HTS) apparatus and method, and subsequent culture and analysis may be performed using a manual or automatic system. In this case, one or more cell types may be used, and at the same time, one or more selected growth factor candidate groups may be used. The cells or selected growth factor candidate groups may be dispensed into the medium as intended by one of ordinary skill in the art who performs the present procedures, or may be randomly dispensed into the tens to thousands of mediums by using an HTS apparatus. When the cell proliferative activity of a particular medium is changed compared to the control, the cells, growth factors and other substances included in the medium are analyzed using the HTS apparatus. One skilled in the art may perform HTS including the procedures described above by using commercialized HTS apparatus and method. Such HTS appratuses may include plates, cell and growth factor dispensers, constant-temperature incubators, automated delivery devices, image analyzers, data storage devices, and data analysis devices, and analysis programs. The degree of influence on cell proliferation can be performed using a quantitation analysis or an image analysis.

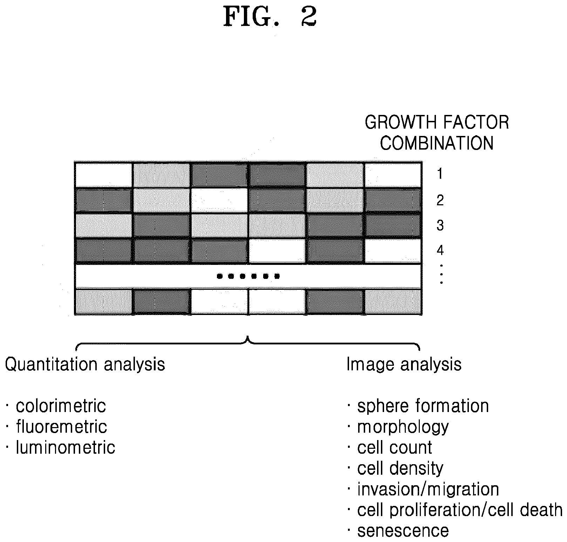

FIG. 2 schematically shows an analysis method for selecting a growth factor combination according to one aspect. In order to measure the degree of influence on cell proliferation, changes in cell proliferation, cell death (apoptosis or necrosis), cell invasion or migration, and changes in cell characteristics (stem cell capacity/differentiation capacity, signaling-path sensitivity) may be measured and determined. The measurement may be performed by a quantitation analysis such as colorimetry, fluorescence measurement, luminescence measurement, or an image analysis using staining such as cell sphere formation, cell morphology, cell count, cell density, invasion/migration, cell proliferation, cell death, or cell senescence. Apparatuses, kits and the like for performing the measurements are commercially available and may be easily used by those skilled in the art according to the manuals provided by the vendors.

In this regard, candidate groups that affect (help) the cell proliferation (z-score value> 0.8) in 70% or more cell samples, may be selected as a first selection condition, that is, candidate groups having a common hit on cell proliferation Also, even when less than 70%, candidate groups having a specific hit on cell proliferation (z-score value> 0.8) may be selected as the second selection condition, that is, candidate groups having specific hit on the cell proliferation. The candidate groups may be growth factor sets including two or more growth factors, or growth factor combination candidate groups. Candidate groups that have a significant effect on cell proliferation, which is confirmed through the measurements, may be registered in a separate growth factor search platform. The term "significance" or "statistical significance" means that there is a statistical probability that can be said that the observed characteristic of the sample is obtained not accidentally but is the actual character of the subject, and whether the result is a significant result may be identified by methods and equations that are known in the art. In this regard, the significance level may be 5%.

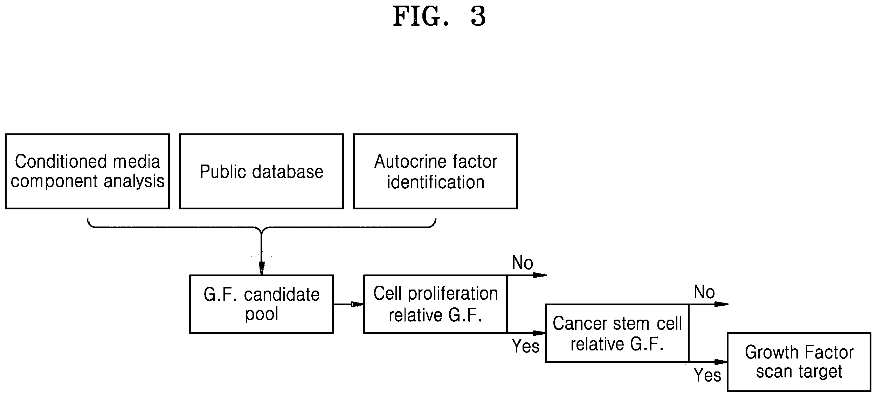

FIG. 3 is a schematic diagram illustrating a process for selecting a growth factor according to an aspect of the present disclosure.

Once the culture medium to help the culture of the cell is identified, the process of selecting a growth factor to be included in the cell culture medium to promote cell culture can be performed. The selecting may include a) analysing of the components contained in the culture medium, b) identifying of information on growth factors affecting cell proliferation published in pathways readily accessible to those skilled in the art, and c) identifying of components autocrined from cells, and finally selecting the growth factors to be added to the cell culture medium.

As described above, when a combination is obtained of desired growth factors selected from a) a growth factor added to the culture medium, b) a known growth factor, and c) an autocrined factor, the cell culture may be performed using a culture medium including the combination. Combinations of growth factors, which have been shown to be involved in cell proliferation, can also be used to determine whether they affect culture of specific cell populations such as cancer stem cells. The combinations of growth factors that have a significant effect on cell proliferation, which was confirmed through the measurements, may be registered in a separate growth factor search platform.

As described above, the growth factor combinations registered in the growth factor search platform may be updated as additional data is collected, and a new condition may be added in addition to the above-described cell or growth factor classification conditions or selection conditions. In addition, growth factor combinations that did not show significant results may be registered in the growth factor search platform as insignificant data. Such insignificant data can be updated as additional data is collected.

Another aspect provides a cell-customized growth factor selecting apparatus for performing the growth factor search.

The apparatus may include, to perform a screening the cell-customized growth factor, an HTS performing apparatus having several tens to hundreds of plates (wells, chips, beads) at the same time, a measuring device capable of performing quantitative or qualitative analysis of cells, and an analytical program capable of performing hit selection, a database and a storage device, including an algorithm for associating and storing a cell type (classification) and a growth factor combination showing a change in cell proliferation rate above a certain level.

### ADVANTAGEOUS EFFECTS OF DISCLOSURE

According to a method of screening a customized growth factor combination for cell culture according to one aspect, the optimal conditions for cell culture can be efficiently selected. Accordingly, the efficiency of cell culture can be maximized.

### BRIEF DESCRIPTION OF DRAWINGS

FIG. 1 is a schematic diagram showing a method of screening a customized growth factor combination for cell culture according to an aspect of the present disclosure.
FIG. 2 schematically shows an analysis method for selecting a growth factor combination according to one aspect.
FIG. 3 is a schematic diagram illustrating a process for selecting a growth factor according to an aspect of the present disclosure.
FIG. 4 shows test results according to concentrations of selected growth factors according to one aspect.
FIG. 5 shows the results of testing the cell count required for growth factor screening.
FIG. 6 shows the test results of measuring the tumor-sphere formation ability of cells to verify the selected growth factor conditions according to one aspect.
FIG. 7 illustrates the reproducibility of a growth factor screening method according to an aspect of the present disclosure.
FIG. 8A shows that selected growth factors statistically significantly increase cell proliferation, according to one aspect.
FIG. 8B shows that the effects of selected growth factors are confirmed by tumor-sphere formation ability results, according to an aspect.
FIG. 9A illustrates selecting cells that are differently affected in the same growth factor conditions, by using a growth factor screening method according to one aspect of the present disclosure.
FIG. 9B shows that the cells affected differently for the same growth factor conditions experience changes in intracellular signal transduction pathways.

### MODE OF DISCLOSURE

Hereinafter, the present disclosure will be described in more detail with reference to the following examples. However, these examples are provided for illustratively describing present disclosure, and the scope of present disclosure is not limited to these examples.

### Example 1. Selecting of growth factors affecting proliferation of tumor spheroids

According to the guidelines of the Institutional Review Board of Samsung Medical Center, cancer tissues were collected after written consent from cancer patients.

After separating the single cells from the cancer tissues, the cells were dispensed in the culture medium of 384-well plates (Neurobasal A medium, N2 Supplement (0.5X - 1X), B27 Supplement w/o vitA (0.5X -1X), L-glutamine 200 mM), and by adding single or combined addition of growth factors of EGF, FGF, HGF, IGF1, IL6, MDK, NRG1, PEDF, PDGF, PIGF, SEMA3A, SHH, TGFB1, RSPO1, WNT3A, NOG, A2M, ANGPT2, CHI3L1, FN1, LGALS1, IGFBP2, TNC, CLU, GPC3, THBS1, CST3, LAMA4, LAMC1, PTGDS, IGFBP7, COL6A1, CTSB, ICAM2, CSK, MMP7, S100A10, SELP, and Laminin, the cellular proliferative activity, morphology, and various molecular changes of cells were evaluated according to each condition. At this time, the growth factor used for the culture-condition selection was determined according to the process specified in FIG. 3 (hereinafter, a process for screening an appropriate culture composition by measuring the cell responses on the single or combined condition of the growth factors will be referred to as GFSCAN.)

The growth factor single/combination that causes cell proliferation or tumor-sphere formation ability or improvement of early cell characteristic reflection ability in 70% or more cell samples of the GFSCAN results, was selected as a common hit, and growth factor combinations, which do not correspond to the common hit, but help cell proliferation, tumor-sphere formation, or the improvement of cell characteristic reflection ability in specific cells, were classified as cell-specific hit. Common hits and specific hits can be selected depending on a subject to be applied, and may be used for cell culture.

### Example 2. Growth factor concentration adequacy test

The concentration adequacy of the growth factor combinations mounted on GFSCAN was tested with respect to 10 types of tumor spheroids. Specifically, the growth factor combinations registered in Example 1 were added to the cell culture medium at a concentration of 0.2 ng/ml to 125 ng/ml (0.2 ng/ml, 1 ng/ml, 5 ng/ml, 25 ng/ml, and 125 ng/ml) to measure the effects on the cell proliferation.

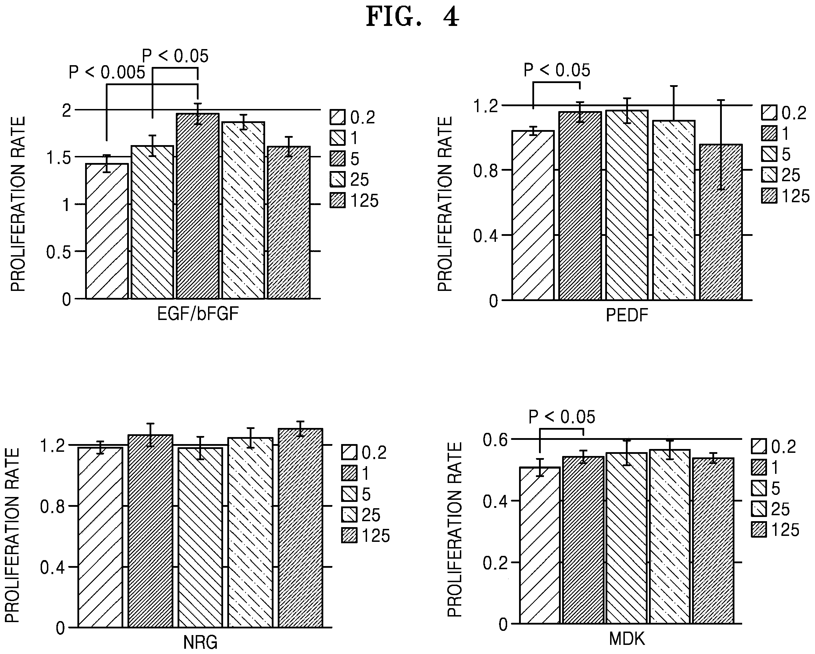

As shown in FIG. 4, in the case of EGF/bFGF, which is a basic growth factor condition, on average, the effects on cell proliferation were greatest at the concentration of 5 ng/ml, and when the growth factor was further added, the cell proliferative activity was not improved. In addition, regarding the improvement of proliferative activity according to the concentration of a growth factor in PEDF, NRG, and MDK among the growth factors mounted on the GFSCAN, the additional effect according to the amount of growth factor added did not occur at the concentration of 1 ng/ml or more. Rather, in the case of PEDF and MDK, the added growth factors were found to have a negative effect on cell proliferation.

### Example 3. Initial cell count adequacy test

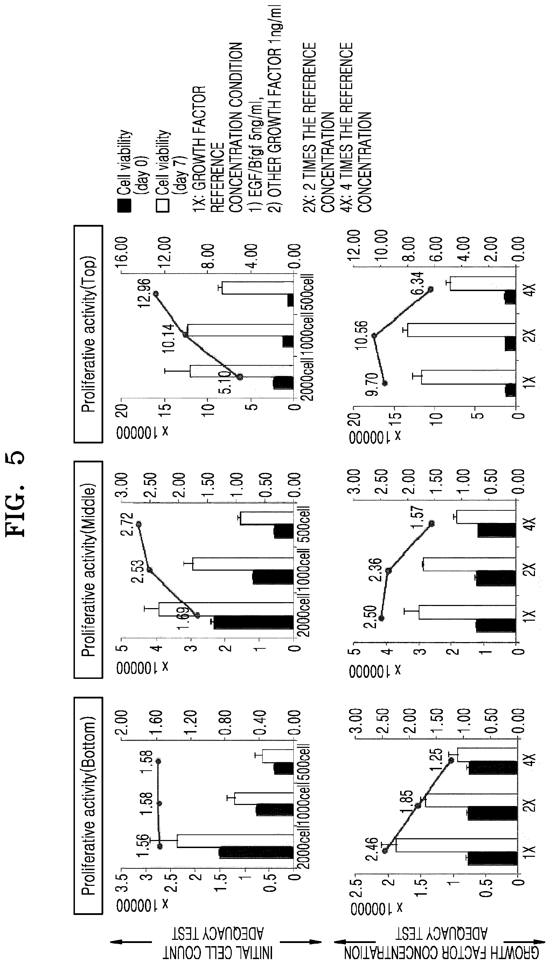

An experiment was conducted to determine the cell count initially dispensed at the time of growth-factor screening. 500 to 2000 cells (500, 1000, or 2000 cells) were dispensed to a plate, and 5 ng/ml EGF/bFGF or any growth factor combination (1 ng/ml) registered in GFSCAN was added thereto, wherein these concentrations were set as a reference concentration. The cell count of grown cells after 7 days was measured, and the proliferative activity was defined as a value obtained by dividing cell viability after 7 days by cell viability on 0 day, and calculated. In order to confirm the effect of the proliferation rate of the cell itself on the GFSCAN results and the optimization conditions, cells having different cell-proliferation rates in three stages (step 1: 0.5-2, stage 2: 2-5, and stage 3: 5) were selected and the initial cell count of each cell was controlled to 500, 1000, and 2000, and the GFSCAN was performed. The obtained results were compared with results obtained under the conventional conditions. As shown in the upper table of FIG. 5, it was confirmed that there were differences in 1) the proliferative activity of cells, and 2) the difference in the cell proliferative activity after 7 days according to the initial cell count. In all cell proliferative activity conditions, commonly, the initial cell count at which the difference in the cell proliferative activity after 7 days is maximized was found to be 500 (top of FIG. 5).

In addition, after verifying the proper concentration condition of the growth factor mounted on GFSCAN at the initial cell count of 500 (1X: reference concentration; 2X: 2 times the reference concentration; and 4X: 4 times the reference concentration), it was confirmed that the proliferative activity was maximized at the reference concentration (1X) condition or reference concentration (2X) condition (bottom of FIG. 5).

### Example 4. GFSCAN data adequacy test

To verify the culture conditions selected through GFSCAN, it was determined whether growth factor combinations were appropriate data with actual cell proliferation effects. To this end, after dispensing a certain number of cells (1, 5, 10, 20, 50, 100, 200, 400) of three tumor spheroids, the tumor-sphere formation ability of cells was compared in a no proliferation condition and a growth factor combination condition, wherein the no proliferation condition was verified by GFSCAN as having no influence on the cell proliferation, and the growth factor combination condition was verified by GFSCAN as helping the cell proliferation.

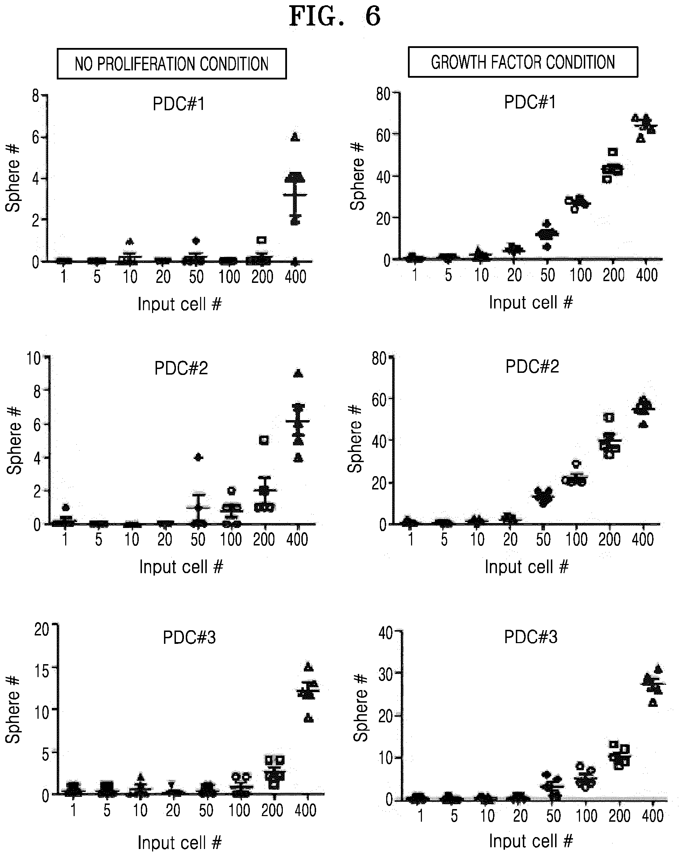

As a result, as shown in FIG. 6, compared to the no proliferation condition, the growth factor combination verified by GFSCAN as helping the cell proliferation [Neurobasal A medium, N2 Supplement(0.5X), B27 Supplement w/o vit A(0.5X), L-glutamine 200 mM(Thermo fisher Inc.), and EGF 20 ng/mℓ and bGFG 20 ng/mℓ (R & D Biosystems)] showed up to 10-fold increase in tumor-sphere formation ability. Accordingly, it was verified that growth factor conditions promoting cell proliferation and tumor-sphere formation can be obtained according to the present disclosure.

### Example 5. Reproducibility test of GFSCAN data

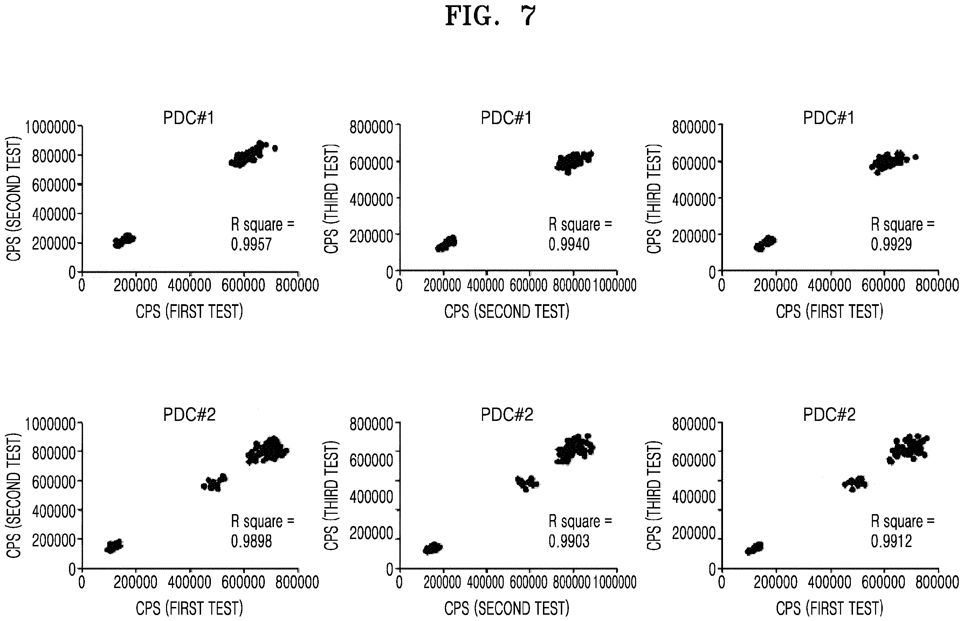

The reproducibility of the data stored in GFSCAN was evaluated. Three experiments (repeated test cycle was one week) were repeatedly performed using randomly extracted growth factor combinations and two tumor spheroids. As a result, as shown in FIG. 7, the correlation coefficient value, (R₂) of the respective experiments was 0.9898 to 0.9957. Accordingly, it was verified that the reproducibility was high.

### Example 6. Effectiveness test of GFSCAN data

By applying GFSCAN to 20 types of brain tumor spheroids, the cell proliferative activity was evaluated under the optimal conditions for each cell selected through GFSCAN (EGF/bFGF 5 ng/ml + SHH/PEDF 1 ng/ml) compared to the conventional growth factor conditions (EGF/bFGF 20 ng/ml).

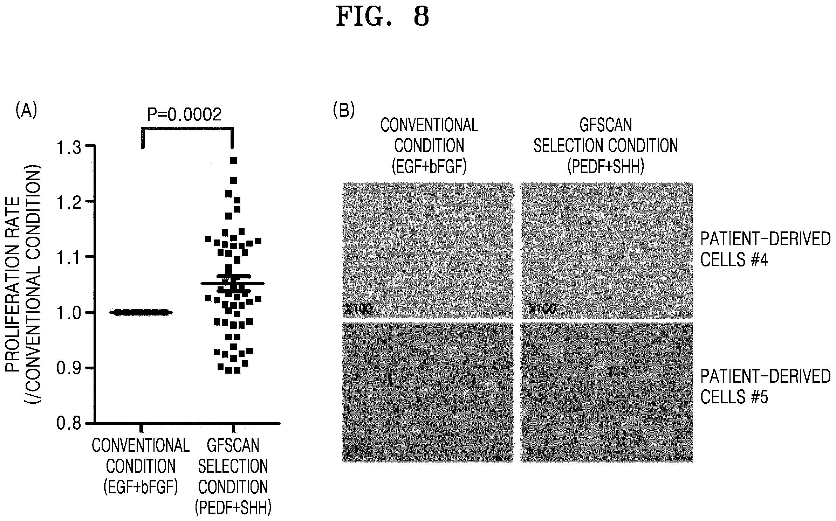

As shown in FIG. 8, it was confirmed that the growth factor combinations of the GFSCAN data significantly increased the cell proliferative activity (a). At this time, with respect to two tumor spheroids, tumor culture proceeded under the conventional growth factor condition and the condition selected through GFSCAN to confirm cell morphology change and tumor-sphere formation ability. It was confirmed that in the novel condition selected through GFSCAN, cell proliferation or tumor-sphere formation ability was improved compared to the conventional condition.

### Example 7. Discovery of growth factor conditions with specific cell proliferation effects through GFSCAN

GFSCAN was performed on 20 tumor spheroids to determine whether there is a difference in the proliferative activity in the identical growth factor condition according to characteristics of the individual patients. Among the GFSCAN conditions, one representative condition (EGF/bFGF 5 ng/ml + TGF-beta 1 ng/ml) was selected, and tumor spheroids with enhanced or inhibited cellular proliferative activity were selected, and GFSCAN results were verified by confirming that the cell proliferative activity was the same as GFSCAN results according to the presence or absence of TGF-beta, through respective cell experiments.

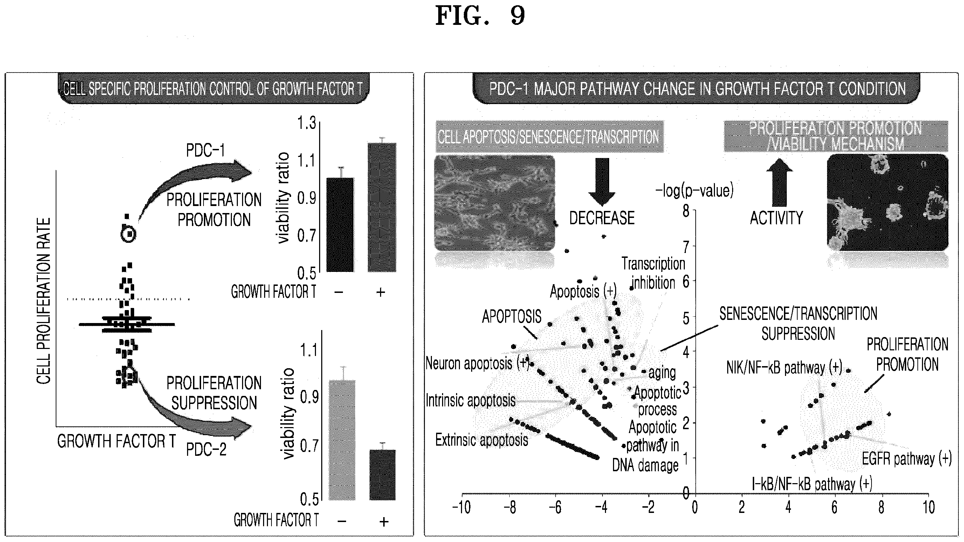

Also, when cultured in cells with improved cell proliferative activity, when TGF-beta was added, signal transduction pathways associated with the promotion of proliferation were activated, or related factors were overexpressed, and the pathways of signal transduction suppressing apoptosis and cell senescence or transcription and related molecules were found to be decreased. (FIG. 9B).

## Claims

1. A method of screening a customized growth factor combination for cell culture, the method comprising:
adding at least one growth factor and cells isolated from a subject to a culture medium;
culturing the cells; and
measuring proliferative activity of the cells.

2. The method of claim 1, wherein the cells are isolated from a disease tissue.

3. The method of claim 2, further comprising classifying the cells according to at least one condition selected from derived subject, derived tissue, disease type, disease severity, or genetic information.

4. The method of claim 3, wherein the growth factor is selected from a list predicted to increase the proliferation rate of cells from an omics-based database according to the classifying of the cells.

5. The method of claim 1, wherein the culturing and the measuring are performed by high-throughput screening (HTS).

6. The method of claim 1, wherein the measuring is performed by quantitative analysis and image analysis.

7. The method of claim 6, wherein the quantitative analysis is performed by using any one of colorimetry, fluorescence measurement, or luminescence measurement.

8. The method of claim 6, wherein the image analysis is analysis of any one of cell sphere formation, cell morphology, cell count, cell density, invasion/migration, cell proliferation, cell death, or cell senescence.

9. The method of claim 1, further comprising, in the measuring, specifying a medium of which cell proliferative activity is changed compared to a control(z-score value > 0.8).

10. The method of claim 9, further comprising, in the measuring of the cell proliferative activity, selecting, as a first selection condition, growth factors that affect cell proliferation (z-score value> 0.8) in 70% or more of cell samples.

11. The method of claim 9, further comprising, in the measuring of the cell proliferative activity, selecting, as a second selection condition, growth factors that affect cell proliferation (z-score value> 0.8) in less than 70% of cell samples.

12. The method of claim 11, further comprising selecting a growth factor, which is selected by the second selection condition, as a growth factor that has a specific effect with respect to a corresponding cell sample.

13. The method of claim 9, further comprising analyzing a growth factor included in the medium.

14. The method of claim 13, further comprising storing a growth factor included in the medium as a selected growth factor in a data storage.

15. The method of claim 14, further comprising storing a growth factor included in media other than the medium as an unselected growth factor in the data storage.

16. A method of screening a cell-customized cell culture condition, the method comprising:
a) adding at least one growth factor and cells isolated from a subject to a culture medium;
b) culturing the cells;
c) measuring cell proliferative activity of the cells;
d) specifying a medium of which the cell proliferative activity is changed compared to a control(z-score value > 0.8) in the measuring,;
e) storing a growth factor included in the medium as a selected growth factor in a data storage;
f) repeatedly performing the processes of a) through d) using the growth factor stored in the process e); and
g) updating pre-stored data using data obtained in process f).

17. The method of claim 16, wherein the process f) is performed by adding growth factors other than the growth factor of process a).

18. The method of claim 16, wherein the process f) is performed by adding cells other than the cells of process a).

19. The method of claim 16, wherein the measuring is performed by quantitative analysis and image analysis.

20. The method of claim 19, wherein the quantitative analysis is performed by using any one of colorimetry, fluorescence measurement, or luminescence measurement.

21. The method of claim 19, wherein the image analysis is analysis of any one of cell sphere formation, cell morphology, cell count, cell density, invasion/migration, cell proliferation, cell death, or cell senescence.
